# EUROPÄISCHE PATENTSCHRIFT

(11) **EP 1 213 991 B1**
(45) Veröffentlichungstag und Bekanntmachung des Hinweises auf die Patenterteilung: **04.06.2003**
(21) Anmeldenummer: 00964227.3
(22) Anmeldetag: 22.09.2000
(51) Int. Cl.: A61B 3/16, A61B 5/00, A61F 2/16

(54) **VORRICHTUNG ZUM MESSEN VON PHYSIKALISCHEN GRÖSSEN, INSBESONDERE ZUR DRUCKMESSUNG IM AUGE**
DEVICE FOR MEASURING PHYSICAL QUANTITIES, ESPECIALLY FOR MEASURING PRESSURE IN THE EYE
DISPOSITIF POUR MESURER DES GRANDEURS PHYSIQUES, NOTAMMENT POUR MESURER LA PRESSION INTRA-OCULAIRE

(30) Priorität: 24.09.1999 DE 19945879
(43) Veröffentlichungstag der Anmeldung: 19.06.2002
(73) Patentinhaber: Acritec Gesellschaft für Ophthalmologische Produkte MbH, 16548 Glienicke (DE); Mesotec Gesellschaft für Medizinische Sensortechnik mbH, 30165 Hannover (DE)
(72) Erfinder: KREINER, Christine, 81545 München (DE); BÖDECKER, Volker, 30165 Hannover (DE); SCHNAKENBERG, Uwe, 52074 Aachen (DE); ULLERICH, Stella, Marianne, 52066 Aachen (DE); WALTER, Peter, 50354 Hürth (DE)
(74) Vertreter: Nöth, Heinz
(86) Internationale Anmeldenummer: EP0009301
(87) Internationale Veröffentlichungsnummer: WO01021063

(56) Entgegenhaltungen:
- EP-A- 0 908 756
- DE-A- 19 858 172
- DE-C- 19 728 069
- US-A- 4 816 031
- US-A- 5 005 577
- PUERS R: "CAPACITIVE SENSORS: WHEN AND HOW TO USE THEM" SENSORS AND ACTUATORS A,CH,ELSEVIER SEQUOIA S.A., LAUSANNE, Bd. A37/38, 1. Juni 1993 (1993-06-01), Seiten 93-105, XP000411381 ISSN: 0924-4247

## Beschreibung

Die Erfindung betrifft eine Vorrichtung nach dem Oberbegriff des Patentanspruches 1, wie aus der DE 197 28 069 C1 bekannt.

Die bekannte Vorrichtung dient zum Messen des Augeninnendrucks und besitzt ein faltbares Implantat, an welchem außerhalb des Blickfeldes des Auges ein telemetrisches System mit einem Drucksensor und einer eine Spule aufweisenden Sendeeinrichtung vorgesehen sind. Mit der Sendeeinrichtung können den Sensorsignalen entsprechende Informationen drahtlos an eine außerhalb des Auges angeordnete Empfangseinrichtung weitergegeben werden. In einer an die Empfangseinrichtung angeschlossenen Auswerteeinrichtung werden die empfangenen Informationen in wiedergebbare Daten umgewandelt.

Bei der bekannten Vorrichtung kann die in das Auge implantierbare Fernmesseinrichtung einen Datalogger aufweisen, in welchem die vom Drucksensor kontinuierlich gelieferten Messdaten speicherbar und aus welchem die Messdaten beim Sendeempfangsbetrieb zeitlich begrenzt bei Bedarf abgefragt werden können.

Eine weitere implantierbare Femmesseinrichtung ist aus "Capacitive sensors: when and how to use them", Puers R., Sensors And Actuators A, Vol. 37-38, Seiten 93-105, bekannt. Dieses Dokument offenbart einen ringförmigen Träger für eine Spule und elektronische Bauelemente, wobei der Träger aus Glas ausgebildet ist.

Aufgabe der Erfindung ist es eine Vorrichtung der eingangsgenannten Art zu schaffen, welche bei hervorragender Empfangsund Sendequalität falt- bzw. rollbar ist.

Diese Aufgabe wird erfindungsgemäß durch die kennzeichnenden Merkmale des Patentanspruches 1 gelöst, wobei die Unteransprüche vorteilhafte Weiterbildungen der Erfindung beinhalten.

Bei der Erfindung ist auf einem faltbaren Träger, insbesondere einer Trägerfolie die Spule in einer ebenen Fläche in Form mehrerer nebeneinander liegender Spulenwindungen, planar aufgebracht. Die die Elektronik und/oder den Sensor enthaltende Fernmesseinrichtung sind bevorzugt in wenigstens einem elektronischen Baustein (Chip) enthalten und ebenfalls mit elektrischer Kontaktierung zu der Spule auf dem faltbaren Träger aufgebracht. Diese Anordnung ist in ein faltbares biokompatibles Implantatmaterial, insbesondere aus Polyorganosiloxan, z.B. Polydimethylsiloxan eingegossen. Hierbei kann das Implantatmaterial nicht nur als Umhüllung für die Sendeeinrichtung und Fernmesseinrichtung, sondern auch als Übertragungsmedium für die zu messende physikalische Größe, welche insbesondere der Augeninnendruck oder auch die Temperatur im Auge sein kann, zum Sensor hin dienen. D.h. bei einer bevorzugten Ausführungsform ist auch der Sensor von dem biokompatiblen Implantatmaterial umgeben. Es ist jedoch auch möglich, den Sensor an einer Sensorfläche, welche gegenüber der zu messenden bzw. zu erfassenden physikalischen Größe empfindlich ist, bzw. in einem bestimmten Sensorbereich freizulegen. Die im Auge vorliegende zu messende physikalische Größe, beispielsweise der Augeninnendruck oder die Temperatur, wirkt dann unmittelbar auf die Sensorfläche oder diesen Sensorbereich. Ferner ist es möglich, ein anderes Übertragungsmedium für die physikalische Größe zu verwenden als das Implantatmaterial.

Durch die planare Ausbildung der Spule mit mehreren nebeneinander liegenden Spulenwindungen, welche bevorzugt in einer zur optischen Achse des Auges bzw. des als Intraokularlinse ausgebildeten Implantats senkrechten Ebene liegt, wird eine hohe Sende- und Empfangsqualität erreicht, ohne dass die Falt- bzw. Rollbarkeit des Implantatmaterials beeinträchtigt wird. Ferner wird für die gesamte Vorrichtung die erforderliche Verträglichkeit mit dem Auge erreicht. Neben einer planaren Lage können auch mehrere planare übereinanderliegende Lagen (Ebenen) für die Spulenwindungen vorgesehen sein.

In bevorzugter Weise ist das Implantat als Intraokularlinse ausgebildet, wobei die Telemetrieeinrichtung und die die Spule aufweisende Sendeeinrichtung außerhalb des optischen Linsenteils, insbesondere im wesentlichen im Bereich der Haptik der Intraokularlinse, welche den optischen Linsenteil umgibt, untergebracht sind. Hierzu kann die Haptik einen den optischen Linsenteil umgebenden ringförmigen Bereich aufweisen, innerhalb welchem die planare Anordnung der Spulenwindungen, untergebracht ist. Die Spulenwindungen sind bevorzugt als planare elektrische Leiterbahnen ausgebildet, die bevorzugt aus Edelmetall, insbesondere Gold bestehen. Die Leiterbahnen der Spulenwindungen werden auf der Trägerfolie in herkömmlicher Planartechnik, beispielsweise durch Metallabscheiden, insbesondere galvanische Abscheidung, wie sie bei Mikrostrukturierungsverfahren bekannt sind, hergestellt.

Das Implantat kann auch ringförmig ausgebildet sein. Die Spulenwindungen sind dann auf wenigstens einer der Ringoberflächen angeordnet. Vorzugsweise erfolgt die Fixation des ringförmigen Implantats am Sulcus des Auges. Der Ring kann zum Teil von einem harten Material, insbesondere PMMA und zum Teil von einem flexiblen Material, insbesondere Silikon gebildet werden. Das Implantat ist vorzugsweise mit einem biokompatiblen Material, beispielsweise Silconkautschuk umhüllt. Der Ring kann auch ganz aus Silikon bestehen, wobei eine stabilisierende Haptik, insbesondere aus PMMA oder einem anderen starren Material vorgesehen ist.

Die Trägerfolie ist als dünne flexible und faltbare Folie ausgebildet, die eine gute Haftung für das Metall der Spulenwindungen gewährleistet, insbesondere besitzt das Folienmaterial dielektrische Eigenschaften und kann aus einem geeigneten Kunststoff, z.B. einem Polyimid, bestehen.

Aufgrund der extremen Roll- bzw. Faltbarkeit der Vorrichtung kann diese ohne die üblichen minimal-invasiven Operationsmethoden zu ändern, in das Auge implantiert werden. Hierdurch können mikroelektronische und sensorische Komponenten für die drahtlose Energie- und Signalübertragung, beispielsweise in Form einer künstlichen Intraokularlinse, welche faltbar ist, in das Auge appliziert werden. Nach der Implantation entfaltet sich die Intraokularlinse.

Anhand der Figuren wird an einem Ausführungsbeispiel die Erfindung noch näher erläutert. Es zeigt:
- Figur 1: eine Draufsicht auf ein als Intraokularlinse ausgebildetes Ausführungsbeispiel;
- Figur 2: in Draufsicht eine Ausführungsform für ein telemetrisches System, welches bei dem in der Figur 1 dargestellten Ausführungsbeispiel zur Anwendung kommen kann;
- Figur 3: eine schnittbildliche Darstellung des in Figur 2 dargestellten telemetrischen Systems;
- Figur 4: eine schnittbildliche Darstellung eines telemetrischen Systems eines weiteren Ausführungsbeispiels;
- Figur 5: ein Ausführungsbeispiel für eine ringförmiges Implantat;
- Figur 6: ein weiteres Ausführungsbeispiel für ein ringförmiges Implantat mit geschlossenen Haptikschlaufen; und
- Figur 7: ein Ausführungsbeispiel für ein ringförmiges Implantat mit offenen Haptikschlaufen.

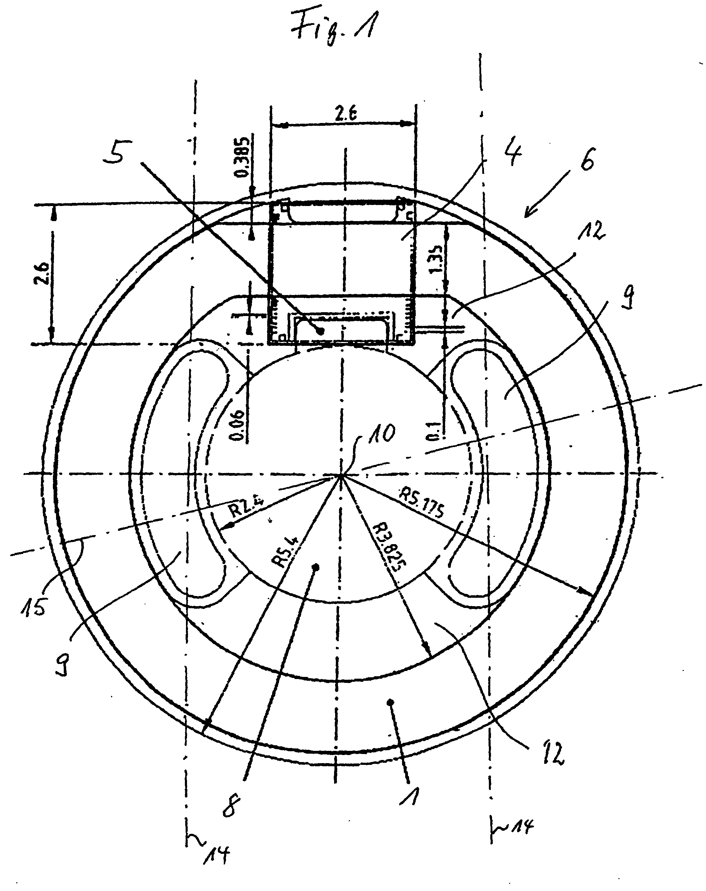

Das dargestellte Ausführungsbeispiel eines Augenimplantats 6 ist als Intraokularlinse ausgebildet. Diese besitzt einen optischen Linsenteil 1, welcher im Sehbereich des Auges einsetzbar ist. Der optische Linsenteil 8 besitzt eine optische Achse 10, welche im wesentlichen senkrecht zur Zeichenebene der Figur 1 verläuft. Die optische Achse ist im implantierten Zustand im wesentlichen zur Sehachse des Auges ausgerichtet. Der optische Linsenteil 8 deckt im wesentlichen das Blickfeld des Auges ab.

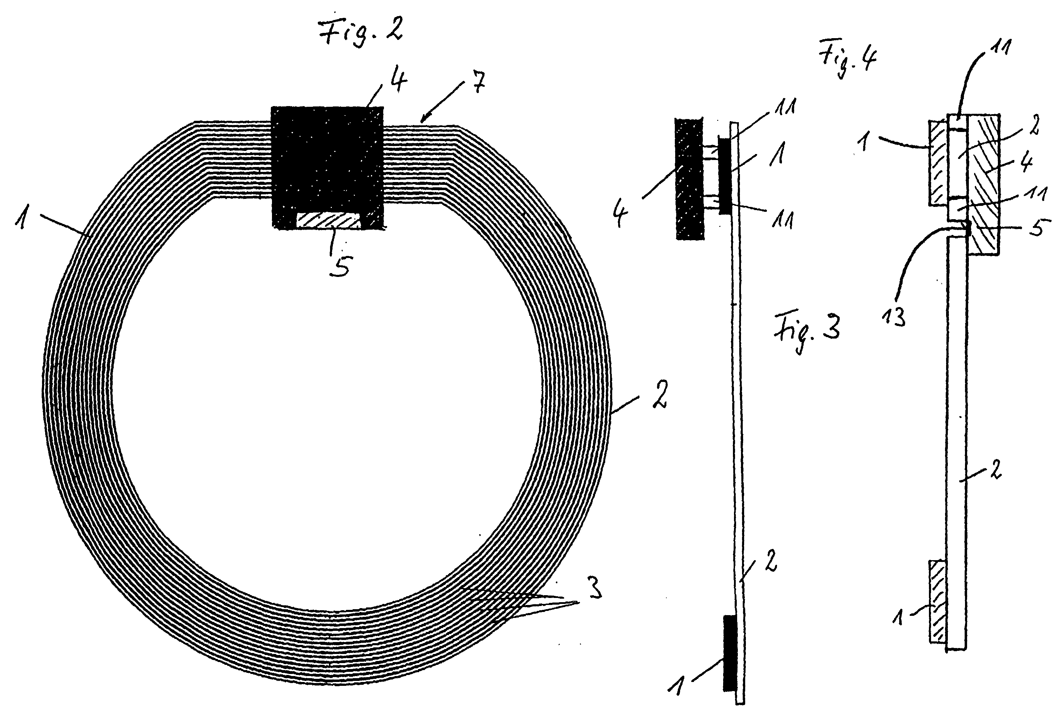

Auf einem als Trägerfolie 2 (Figur 2), ausgebildeten ringförmigen Träger welcher flexibel d. h. faltbar und rollbar ausgebildet ist, befindet sich eine Spule 1, welche in der Sende- und Empfangseinrichtung die Induktivität bildet. Die Spule wird von planaren Spulenwindungen 3, in Form von nebeneinander liegenden Leiterbahnen gebildet. Die Leiterbahnen der Spulenwindungen 3 liegen nebeneinander in einer im wesentlichen senkrecht zur optischen Achse 10, verlaufenden Ebene. Die Breite einer Spulenwindung liegt in der Größenordnung von ca. 3 bis 90 µm, vorzugsweise von ca. 10 bis 90 µm. Es können etwa 10 bis 65 Spulenwindungen in einer jeweiligen Ebene für die Spule 1 vorgesehen sein. Durch eine derartige Ausbildung der Spule 1 bleibt die Faltbarkeit, Rollbarkeit und gegebenenfalls Knickbarkeit der Trägerfolie 2 unbeeinträchtigt. Die Spulenwindungen 3 können beispielsweise durch galvanische Abscheidung, wie sie bei Verfahren der Mikrostrukturierung bekannt, ist, hergestellt werden. Beim dargestellten Ausführungsbeispiel befindet sich die Spule 1 auf einer kreisringförmigen Fläche. In Anpassung an den Einsatzort des Implantates 6 kann die Spule jedoch auch oval, oval-ähnlich ausgebildet sein oder eine andere Ausgestalltung haben.

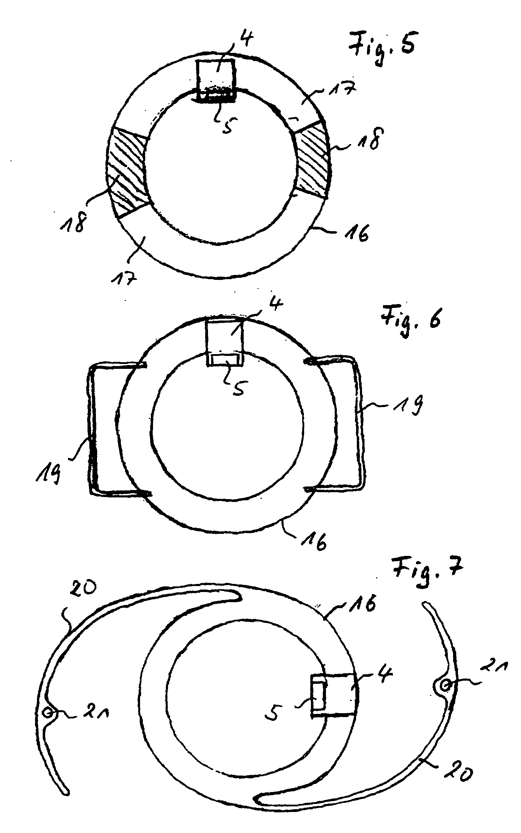

Auf der Trägerfolie 2 befindet sich ferner die Elektronik des telemetrischen Systems, welche in einem elektronischen Baustein (Chip) 4 untergebracht ist, wobei selbstverständlich auch mehrere elektronische Bausteine Verwendung finden können. An diesem elektronischen Baustein 4 kann bevorzugt in einem Randbereich ein Sensor 5 zur Erfassung der zu messenden physikalischen Größe, insbesondere des Augeninnendrucks vorgesehen sein. Wie die Figur 3 zeigt, ist der elektronische Baustein 4 in geeigneter Weise mit der Spule 1 kontaktiert (elektrische Kontaktierungen 11). Im Bereich des elektronische Bausteins 4 verlaufen zur erleichterten Kontaktierung die Spulenwindungen 3 vorzugsweise im wesentlichen geradlinig, wie es in einem geradlinigen Windungsbereich 7 der Figur 2 gezeigt ist. Die elektrische Kontaktierung 11 zwischen der Spule 1 und dem elektronischen Bauteil 4 kann in Hybrid- oder Flip-Chip-Technologie durch Bonden erreicht werden. Die elektrischen Kontaktierungsstellen 11 (Figur 3) können durch Goldbumps mit einer Dicke von 30 µm und weniger gebildet werden. Neben monolithischer Bauform kann der Chip bzw. können die elektronischen Bausteine in eine oder mehrere Folien eingearbeitet und somit falt- bzw. rollbar sein.

Die planaren Spulenwindungen besitzen eine Dicke (Höhe) im Bereich von 5 bis 60 µm. Die Höhe des elektronischen Bausteins 4 beträgt ca. 600 µm und kann wesentlich geringer beispielsweise 300 µm betragen. Die Fläche des elektronischen Bausteins 4 beträgt ca. 2,0 mm x 2,0 mm. Die Dicke der Trägerfolie kann etwa 8 µm betragen. Die Spule kann einen Außenradius von ca. 5,15 mm und einen Innenradius von ca. 3,85 mm aufweisen. Der Bereich der Trägerfolie 2, welcher innerhalb der Spule 1 liegt, kann ausgestanzt sein, so dass die Trägerfolie 2 als ringförmige Trägerfolie, welche im wesentlichen mit den Spulenwindungen 3 bedeckt ist, vorliegt.

Die Trägerfolie 2 mit den darauf angeordneten telemetrischen Einrichtungen, wie sie in den Figuren 2 und 3 gezeigt sind, wird von einem biokompatiblen Implantatmaterial, insbesondere Linsenmaterial vollständig, insbesondere durch Eingießen umhüllt. Das Implantatmaterial bzw. Linsenmaterial überdeckt auch den Sensor 5, welcher insbesondere als Drucksensor ausgebildet ist. Die Figur 1 zeigt die Intraokularlinse, in welche das in den Figuren 2 bis 4 dargestellte telemetrische System eingegossen ist. Die in der Figur 1 wieder gegebenen Abmessungsangaben sind beispielhafte Angaben, welche innerhalb der für eine Augenimplantation zulässigen Grenzen variierbar sind.

Wie aus der Figur 1 zu ersehen ist, befindet sich die Spule 1 innerhalb eines ringförmigen Haptikbereiches, welcher den optischen Linsenteil 8 konzentrisch umgibt. Es kann sich um einen Kreisring oder ovalen oder ovalähnlichen Ring handeln. Ein zwischen diesem ringförmigen Haptikbereich und dem optischen Linsenteil 8 liegender ringförmiger Bereich 12 des Linsenmaterials ist mit Langlöchern 9 versehen, die an ihren Begrenzüngsrändern etwa konzentrisch zu der ringförmigen Spule 1 und dem ringförmigen Bereich 12 um die optische Achse 10 sich erstrecken. Diese Langlöcher 9 erleichtern nicht nur das Falten bzw. Rollen der Linse, sondern unterstützen die Fixierung der Linse im Auge, da in diese Langlöcher Augengewebe einwachsen kann. Wie aus der Figur 1 ferner zu ersehen ist, befindet sich der Sensor 5 in der Nähe des optischen Linsenteiles 8. Er liegt zwischen dem optischen Linsenteil 8 und dem Innenrand der Spule 1 in einem Bereich, welcher die Fläche der Spule 1 nicht überlappt. Der Sensor 5 wird von einem Linsenmaterial umschlossen, das sich zwischen zwei Enden der Langlöcher 9 im ringförmigen Bereich 12 des Linsenmaterials befindet. Das Linsenmaterial dient zur Übertragung der im Auge zu messenden physikalischen Größe, beispielsweise der Temperatur oder des Augeninnendrucks. In bevorzugter Weise kommt ein Polyorganosiloxan, insbesondere Polydimethylsiloxan für das Linsenmaterial zur Anwendung. Es ist auch möglich, ein anderes Übertragungsmedium im Bereich des Sensors 5 oder eines auf die physikalische Größe (z.B. Druck, Temperatur) ansprechenden Sensorbereichs vorzusehen oder diesen Bereich freizulegen, wie es anhand der Figur 4 noch erläutert wird.

Der Außendurchmesser der Intraokularlinse kann etwa 12 mm oder weniger z. B. 8,5 mm betragen. Der Durchmesser des optischen Linsenteils 8 kann 6 mm oder weniger, beispielsweise 4,8 mm betragen. Die Dicke der Linse im Zentrum des optischen Linsenteiles 8 kann etwa 0,780 mm oder weniger betragen. Im nichtoptischen Bereich kann die Dicke 0,500 mm oder weniger betragen, wobei jedoch im Bereich der elektronischen Baueinheit 4 gewährleistet ist, dass diese vom Linsenmaterial vollständig umhüllt ist und dem gemäß die Linse in diesem Bereich eine entsprechende Dicke aufweist. Die Länge der Langlöcher 9 kann etwa 4,6 mm oder geringer bemessen sein. Die Breite kann 1,2 mm oder weniger betragen.

Bei dem in der Fig. 3 dargestellten Ausführungsbeispiel befinden sich die Spule 1 und der elektronische Baustein 4 auf der gleichen Seite der Trägerfolie 2. Bei dem in der Figur 4 dargestellten Ausführungsbeispiel befindet sich die Spule 1 auf der einen Seite der Trägerfolie 2 und der elektronische Baustein 4 auf der anderen Seite der Trägerfolie 2. Die elektrische Kontaktierung 11 zwischen der Spule 1 und dem elektronischen Baustein 4 erfolgt mit Hilfe von Durchkontaktieren durch die Trägerfolie 2.

Wie aus dem Ausführungsbeispiel der Figur 4 zu ersehen ist, kann ein für die zu erfassende physikalische Größe empfindlicher Bereich des Sensors 5 freigelegt sein. Beim dargestellten Ausführungsbeispiel ist es eine Sensorfläche 13. Hierzu kann in der Trägerfolie 2 eine Ausnehmung vorgesehen sein. Diese Ausnehmung befindet sich auch in dem umhüllenden Implantat bzw. Intraokularlinsenmaterial. Es ist jedoch auch möglich, dass in der Ausnehmung ein anderes die physikalische Größe übertragendes Material als das Implantatmaterial verwendet wird. Beim dargestellten Ausführungsbeispiel der Fig. 4 befindet sich die freigelegte Sensorfläche 13 an der Innenseite des Sensors 5. Die freigelegte Sensorfläche kann auch auf der anderen Seite, d.h. an der Außenseite des Sensors 5 liegen.

Wie aus der Fig. 1 zu ersehen ist, kann das Implantat- bzw. Linsenmaterial um etwa parallel zueinander verlaufende Faltkanten 14, gefaltet oder gerollt werden, die beidseits des elektronischen Bausteins 4 liegen. Selbst wenn der elektronische Baustein 4 aus einem nicht faltbaren monolithischen Baustein besteht, erreicht man eine erhebliche Verringerung des Implantatquerschnittes für die Implantation. Die beiden Faltkanten 14 verlaufen beidseits des elektronischen Bausteins. Ferner kann das Implantat auch entlang einer durch die Linsenmitte (optische Achse 10) verlaufende Faltkante 15 gefaltet werden. Hieraus ist ersichtlich, dass es eine große Anzahl an Faltmöglichkeiten des Implantats gibt, selbst wenn der elektronische Baustein 4 monolithisch ausgebildet ist. Durch die spezielle Ausbildung der Spule 1 ist diese unter Erzielung einer hohen Induktivität faltbar.

Im elektronische Baustein 4 kann ein Speicher vorgesehen sein, welcher die vom Sensor, insbesondere Drucksensor 5 kontinuierlich aufgenommenen Druckwerte speichert. Diese Druckwerte können aus diesem Speicher von Zeit zu Zeit beispielsweise im Turnus von einer Woche jeweils abgerufen werden und von der Telemetrieeinrichtung auf eine nicht näher dargestellte Empfangseinrichtung mit angeschlossener Auswerteeinrichtung übertragen werden, wie es beispielsweise in der deutschen Patentschrift DE 197 28 069 C1 beschrieben ist. Es ist auch möglich, dass der elektronische Baustein 4 aus faltbarem Trägermaterial gebildet wird, so dass eine Verformung der Intraokularlinse auf einen geringen Durchmesser möglich ist und am Auge ein nur kleiner Schnitt für die Implantation vorgesehen werden muss. Das Linsenmaterial ist in der Weise ausgebildet, dass es sich nach der Implantation entfaltet und die gewünschte Linsenform annimmt.

Bei den in den Figuren 5 bis 7 dargestellten Ausführungsbeispielen ist ein Implantatkörper 16 ringförmig ausgebildet. Die im Ringinnern vorgesehene Ausnehmung ist mindestens so bemessen, dass sie außerhalb des Blickfeldes liegt, wenn das ringförmige Implantat im Auge angeordnet ist. Die nicht näher dargestellte Spule ist so ausgebildet, wie es in Figur 2 dargestellt ist. Sie befindet sich auf einer oder beiden Oberflächen des ringförmigen Implantats. Der Anschluss des Sensors 5 und des elektronischen Bauteils 4 erfolgt in der gleichen Weise, wie es in den vorherigen Ausführungsbeispielen erläutert wurde. Der Sensor 5 befindet sich innerhalb der ringförmigen Anordnung der Spule 1, wie es aus Figur 2 zu ersehen ist.

Bei dem in Figur 5 dargestellten Ausführungsbeispiel besteht das ringförmige Implantat 16 aus harten bzw. starren Ringteilen 17, vorzugsweise aus PMMA sowie aus flexiblen Ringteilen 18, insbesondere aus Silikon. Hierdurch ist die Faltung des ringförmigen Implantats 16 um eine durch die flexiblen Ringteile 18 gelegte Faltungsachse möglich. Der Außendurchmesser des Ringes beträgt etwa 12 bis 15 mm. Die Breite des Ringes kann 1 bis 3 mm betragen.

Bei dem in der Figur 6 dargestellten Ausführungsbeispiel besitzt der ringförmige Implantatkörper 16 geschlossene Haptikschlaufen 19. Das in Figur 7 dargestellte Ausführungsbeispiel besitzt offene Haptikschlaufen 20. Die ringförmigen Implantatkörper 16 der Ausführungsbeispiele 6 und 7 betsehen vorzugsweise aus Silikonkautschuk. Die Haptikschlaufen 19 und 20 bestehen vorzugsweise aus einem starren Material, insbesondere PMMA. Bei dem in der Figur 7 dargestellten Ausführungsbeispiel sind in den offenen Haptikschlaufen 20 Fixationslöcher 21 vorgesehen. Hierdurch wird eine stabile Positionierung des Implantatkörpers 16 im Auge gewährleistet. Die Ausführungsbeispiele der Figuren 5 bis 7 sind für eine Fixation im Sulcus des Auges geeignet. Gegebenenfalls können auch beim Ausführungsbeispiel der Figur 5 zusätzliche nicht näher dargestellte Fixationslöcher vorgesehen sein.

Die Ausführungsbeispiele der Figuren 5, 6 und 7 können mit einer Umhüllung aus Silikonkautschuk oder einer anderen biokompatiblen Umhüllung vollständig ummantelt sein. Die Übertragung des Augeninnendruckes auf die Sensorfläche des Drucksensors 5 erfolgt über diese nachgiebige Umhüllung. Das Umhüllungsmaterial bildet das Übertragungsmedium des Augeninnendrucks auf die Sensorfläche des Sensors 5.

Man erreicht daher bei allen Ausführungsbeispielen eine vollständige Umhüllung des Implantats mit biokompatiblem Material und eine einwandfreie Druckübertragung auf die Sensorfläche des Sensors 5 über das Umhüllungsmaterial.

### [Bezugszeichenliste]

- 1: Spule
- 2: Trägerfolie
- 3: Spulenwindungen
- 4: elektronischer Baustein (Chip)
- 5: Sensor, insbesondere Drucksensor
- 6: Implantat, insbesondere Intraokularlinse
- 7: geradliniger Windungsbereich
- 8: optischer Linsenteil
- 9: Langloch
- 10: optische Achse
- 11: elektrische Kontaktierung
- 12: ringförmiger Bereich
- 13: Sensorfläche
- 14: Faltkante
- 15: Faltkante
- 16: ringförmiger Implantatkörper
- 17: starres Ringteil
- 18: flexibles Ringteil
- 19: geschlossene Haptikschlaufe
- 20: offene Haptikschlaufe
- 21: Fixationsloch

## Patentansprüche

1. Vorrichtung zum Messen physikalischer Größen im Auge mit einem faltbaren Implantat, an welchem außerhalb eines das Blickfeld des Auges überdeckenden Implantatteil eine Fernmesseinrichtung mit einem Sensor und einer eine Spule aufweisenden Sendeeinrichtung zur drahtlosen Übertragung von den Sensorsignalen entsprechenden Informationen angeordnet ist, und einer außerhalb des Auges angeordneten Empfangseinrichtung, welche die von der Sendeeinrichtung gesendeten Informationen empfängt, und einer Auswerteeinrichtung, welche die empfangenen Informationen in wiedergebbare Daten wandelt, **dadurch gekennzeichnet, dass** auf einem faltbaren ringförmigen Träger (2; 16) die Spule (1) in Form mehrerer nebeneinander liegender Spulenwindungen in wenigstens einer Fläche angeordnet ist und mit der Spule wenigstens ein die Elektronik der Fernmesseinrichtung enthaltender elektronischer Baustein (4) elektrisch kontaktiert ist und dass diese Anordnung in das faltbare biokompatible Implantatmaterial eingegossen ist.

2. Vorrichtung nach Anspruch 1, **dadurch gekennzeichnet, dass** Spulenwindungen (3) aus planaren elektrischen Leiterbahnen gebildet sind.

3. Vorrichtung nach Anspruch 1 oder 2, **dadurch gekennzeichnet, dass** die Spulenwindungen (3) in einer oder mehreren Ebenen angeordnet sind.

4. Vorrichtung nach einem der Ansprüche 1 bis 3, **dadurch gekennzeichnet, dass** der Sensor (5) ganz oder teilweise von einem die physikalische Größe übertragenden Übertragungsmedium umhüllt ist.

5. Vorrichtung nach einem der Ansprüche 1 bis 4, **dadurch gekennzeichnet, dass** das biokompatible Material, mit welchem die Vorrichtung umhüllt ist, dass Übertragungsmedium bildet.

6. Vorrichtung nach einem der Ansprüche 1 bis 5, **dadurch gekennzeichnet, dass** die Spulenwindungen (3) im Bereich ihrer Verbindung mit dem elektronischen Baustein (4) im wesentlichen geradlinig verlaufen.

7. Vorrichtung nach einem der Ansprüche 1 bis 6, **dadurch gekennzeichnet, dass** die Spulenwindungen (3) im wesentlichen sich im gesamten außerhalb des Blickfeldes des Auges liegenden Implantatteil erstrecken.

8. Vorrichtung nach einem der Ansprüche 1 bis 7, **dadurch gekennzeichnet, dass** der Sensor (5) als Drucksensor ausgebildet ist.

9. Vorrichtung nach Anspruch 8, **dadurch gekennzeichnet, dass** der Drucksensor (5) kontinuierlich den Augeninnendruck mißt und die Elektronik der Fernmesseinrichtung einen Speicher aufweist, in welchem die Sensorsignale für ein zeitlich begrenztes Senden an eine Empfangseinrichtung gespeichert werden.

10. Vorrichtung nach einem der Ansprüche 1 bis 9, **dadurch gekennzeichnet, dass** der Sensor (5) in einem die Fläche der Spulenwindungen (3) nicht überlappenden Bereich außerhalb des Blickfeldes des Auges liegt.

11. Vorrichtung nach einem der Ansprüche 1 bis 10, **dadurch gekennzeichnet, dass** der Sensor (5) innerhalb des von der Spule (1) gebildeten Ringes liegt.

12. Vorrichtung nach einem der Ansprüche 1 bis 11, **dadurch gekennzeichnet, dass** das Implantat (6) als Intraokularlinse ausgebildet ist und dass der ringförmige Träger (2) im Bereich des optischen Linsenteils (8) eine Ausnehmung aufweist, welche innerhalb der Spulenwindungen (3) liegt.

13. Vorrichtung nach einem der Ansprüche 1 bis 12, **dadurch gekennzeichnet, dass** im Implantatmaterial zwischen der Spule (1) und dem das Blickfeld durchsetzenden Implantatteil, insbesondere optischen Linsenteil (8) der Intraokularlinse, Langlöchern (9) eingeformt sind.

14. Vorrichtung nach Anspruch 13, **dadurch gekennzeichnet, dass** der Sensor (5) in einem ringförmigen Bereich (12) des Implantatmaterials liegt, in welchem die Langlöcher (9) sich erstrecken.

15. Vorrichtung nach einem der Ansprüche 1 bis 14, **dadurch gekennzeichnet, dass** die Fläche bzw. die Flächen, in welcher bzw. in welchen die Spule (1) angeordnet ist, sich etwa senkrecht zur optischen Achse (10) des als Intraokularlinse ausgebildeten Implantats (6) erstreckt bzw. erstrecken.

16. Vorrichtung nach einem der Ansprüche 1 bis 15, **dadurch gekennzeichnet, dass** die Spule (1) an der einen Oberfläche und der elektronische Baustein (4) auf der anderen Oberfläche des ringförmigen Trägers (2; 16) angeordnet sind.

17. Vorrichtung nach einem der Ansprüche 1 bis 11 und 15, 16, **gekennzeichnet durch** einen ringförmigen Implantatkörper (16) aus zumindest teilweise flexiblen Material, welcher den Träger für die Spule (1) bildet.

18. Vorrichtung nach Anspruch 17, **dadurch gekennzeichnet, dass** der ringförmige Implantatkörper (16) im Sulcus des Auges fixierbar ist.

## Claims

1. A device for measuring physical parameters in the eye, with a foldable implant on which there is arranged, outside an implant portion covering the field of vision of the eye, a telemetry device with a sensor and a transmitting device having a coil for the wireless transmission of items of information corresponding to the sensor signals, and a receiving device which is arranged outside the eye and which receives the items of information sent by the transmitting device, and an evaluation device which converts the received items of information into reproducible data, **characterised in that** the coil (1) is arranged in the form of a plurality of mutually juxtaposed coil windings in at least one surface on a foldable annular carrier (2; 16) and at least one electronic unit (4) containing the electronics of the telemetry device is electrically contacted with the coil and that said arrangement is cast into the foldable biocompatible implant material.

2. A device according to claim 1 **characterised in that** coil windings (3) are formed from planar electrical conductor tracks.

3. A device according to claim 1 or claim 2 **characterised in that** the coil windings (3) are arranged in one or more planes.

4. A device according to one of claims 1 to 3 **characterised in that** the sensor (5) is entirely or partially encased by a transmission medium for transmitting the physical parameter.

5. A device according to one of claims 1 to 4 **characterised in that** the biocompatible material with which the device is encased forms the transmission medium.

6. A device according to one of claims 1 to 5 **characterised in that** the coil windings (3) extend substantially rectilinearly in the region of their connection to the electronic unit (4).

7. A device according to one of claims 1 to 6 **characterised in that** the coil windings (3) extend substantially in the entire implant portion which is outside the field of vision of the eye.

8. A device according to one of claims 1 to 7 **characterised in that** the sensor (5) is in the form of a pressure sensor.

9. A device according to claim 8 **characterised in that** the pressure sensor (5) continuously measures the internal pressure of the eye and the electronics of the telemetry device has a memory in which are stored the sensor signals for time-limited transmission to a receiving device.

10. A device according to one of claims 1 to 9 **characterised in that** the sensor (5) is disposed in a region outside the field of vision of the eye, which region does not overlap the surface of the coil windings (3).

11. A device according to one of claims 1 to 10 **characterised in that** the sensor (5) lies within the ring formed by the coil (1).

12. A device according to one of claims 1 to 11 **characterised in that** the implant (6) is in the form of an intraocular lens and that in the region of the optical lens portion (8) the annular carrier (2) has an opening which lies within the coil windings (3).

13. A device according to one of claims 1 to 12 **characterised in that** slots (9) are formed in the implant material between the coil (1) and the implant portion passing through the field of vision, in particular the optical lens portion (8) of the intraocular lens.

14. A device according to claim 13 **characterised in that** the sensor (5) is in an annular region (12) of the implant material in which the slots (9) extend.

15. A device according to one of claims 1 to 14 **characterised in that** the surface or surfaces in which the coil (1) is arranged extends or extend substantially perpendicularly to the optical axis (10) of the implant (6) which is in the form of an intraocular lens.

16. A device according to one of claims 1 to 15 **characterised in that** the coil (1) is arranged at the one surface of the annular carrier (2; 16) and the electronic unit (4) is arranged on the other surface of the carrier.

17. A device according to one of claims 1 to 11 and 15 and 16 **characterised by** an annular implant body (16) of at least partially flexible material, which forms the carrier for the coil (1).

18. A device according to claim 17 **characterised in that** the annular implant body (16) can be fixed in the sulcus of the eye.

## Revendications

1. Dispositif pour mesurer des grandeurs physiques intraoculaires avec un implant pliable, sur lequel, à l'extérieur d'une partie d'implant couvrant le champ de vision de l'oeil, est disposé un dispositif de télémétrie comportant un capteur et un dispositif émetteur présentant une bobine pour transmettre sans fil des informations correspondant à des signaux de capteur, et un dispositif récepteur disposé à l'extérieur de l'oeil qui reçoit les informations émises par le dispositif émetteur, et un dispositif analyseur qui convertit les informations reçues en données restituables, **caractérisé en ce que** sur un support annulaire (2 ; 16) pliable, la bobine (1) est disposée dans au moins une face sous la forme de plusieurs spires de bobine juxtaposées les unes aux autres et au moins un composant électronique (4) contenant le dispositif de télémétrie électronique est connecté électriquement avec la bobine et que cet agencement est noyé dans le matériau d'implant biocompatible pliable.

2. Dispositif selon la revendication 1, **caractérisé en ce que** des spires de bobine (3) sont constituées de pistes conductrices électriques planes.

3. Dispositif selon la revendication 1 ou 2, **caractérisé en ce que** les spires de bobine (3) sont disposées dans un ou plusieurs plans.

4. Dispositif selon l'une des revendications 1 à 3, **caractérisé en ce que** le capteur (5) est entièrement ou partiellement enveloppé d'un milieu de transmission transmettant la grandeur physique.

5. Dispositif selon l'une des revendications 1 à 4, **caractérisé en ce que** le matériau biocompatible dont est enveloppé le dispositif forme le milieu de transmission.

6. Dispositif selon l'une des revendications 1 à 5, **caractérisé en ce que** les spires de bobine (3) dans la zone de leur liaison avec le composant électronique (4) s'étendent essentiellement de manière rectiligne.

7. Dispositif selon l'une des revendications 1 à 6, **caractérisé en ce que** les spires de bobine (3) s'étendent essentiellement dans la partie d'implant située dans l'ensemble à l'extérieur du champ de vision de l'oeil.

8. Dispositif selon l'une des revendications 1 à 7, **caractérisé en ce que** le capteur (5) est réalisé en tant que capteur de pression.

9. Dispositif selon la revendication 8, **caractérisé en ce que** le capteur de pression (5) mesure la pression de l'oeil en continu et que le système électronique du dispositif télémétrique présente une mémoire, dans laquelle sont stockés les signaux de capteur pour une émission limitée dans le temps à un dispositif récepteur.

10. Dispositif selon l'une des revendications 1 à 9, **caractérisé en ce que** le capteur (5) se situe à l'extérieur du champ de vision de l'oeil dans une zone ne chevauchant pas la surface des spires de bobine (3).

11. Dispositif selon l'une des revendications 1 à 10, **caractérisé en ce que** le capteur (5) se situe à l'intérieur de l'anneau formé par la bobine (1).

12. Dispositif selon l'une des revendications 1 à 11, **caractérisé en ce que** l'implant (6) est réalisé sous forme de lentille intraoculaire et que le support annulaire (2) comporte dans la zone de la partie de lentille optique (8) un évidement qui se situe à l'intérieur des spires de bobine (3).

13. Dispositif selon l'une des revendications 1 à 12, **caractérisé en ce que** des trous oblongs sont ménagés dans le matériau d'implant entre la bobine (1) et la partie d'implant traversant le champ de vision, en particulier dans la partie de lentille optique (8) de la lentille intraoculaire.

14. Dispositif selon la revendication 13, **caractérisé en ce que** le capteur (5) se situe dans une zone annulaire (12) du matériau d'implant dans laquelle s'étendent les trous oblongs (9).

15. Dispositif selon l'une des revendications 1 à 14, **caractérisé en ce que** la surface ou les surfaces dans laquelle ou lesquelles est disposée la bobine (1) s'étend ou s'étendent sensiblement perpendiculairement à l'axe optique (10) de l'implant (6) réalisé en tant que lentille intraoculaire.

16. Dispositif selon l'une des revendications 1 à 15, **caractérisé en ce que** la bobine (1) est disposée sur une face et le composant électronique (4) sur l'autre face du support annulaire (2 ; 16).

17. Dispositif selon l'une des revendications 1 à 11 et 15, 16, **caractérisé par** un corps d'implant annulaire (16) en matériau au moins partiellement souple, lequel forme le support de la bobine (1).

18. Dispositif selon la revendication 17, **caractérisé en ce que** le corps d'implant annulaire (16) peut être fixé dans le sulcus de l'oeil.
